# EUROPÄISCHE PATENTSCHRIFT

(11) **EP 0 992 478 B1**
(45) Veröffentlichungstag und Bekanntmachung des Hinweises auf die Patenterteilung: **05.02.2003**
(21) Anmeldenummer: 99119143.8
(22) Anmeldetag: 05.10.1999
(51) Int. Cl.: C07C 46/10, C07C 46/08, C07C 50/02

(54) **Verfahren zur Reinigung und Isolierung von 2,3,5-Trimethyl-p-benzochinon**
Process for the purification and isolation of 2,3,5-trimethyl-p-benzoquinone
Procédé pour la purification et l' isolation de 2,3,5-triméthyl-p-benzoquinone

(30) Priorität: 06.10.1998 DE 19846003
(43) Veröffentlichungstag der Anmeldung: 12.04.2000
(73) Patentinhaber: BASF AKTIENGESELLSCHAFT, 67056 Ludwigshafen (DE)
(72) Erfinder: Ruff, Detlef, Dr., 67067 Ludwigshafen (DE); Baumann, Dieter, 69190 Walldorf (DE); Aquila, Werner, Dr., 68309 Mannheim (DE); Schubert, Jürgen, Dr., 67246 Dirnstein (DE); Lechtken, Peter, Dr., 67227 Frankenthal (DE)
(74) Vertreter: Isenbruck, Günter, Dr.

(56) Entgegenhaltungen:
- EP-A- 0 216 351
- FR-A- 2 138 030
- FR-A- 2 268 779

## Beschreibung

Die vorliegende Erfindung betrifft ein Verfahren zur Reinigung von 2,3,5-Trimethyl-p-benzochinon (nachstehend als TMC bezeichnet) und zur Isolierung von TMC aus einem TMC enthaltenden Gemisch.

TMC ist ein wichtiges Zwischenprodukt für die Herstellung von Trimethylhydrochinon, einem essentiellen Vorprodukt für die Synthese von α-Tocopherol (Vitamin E). Vor dem Hintergrund der hohen Bedeutung von Vitamin E wurden bereits einige Verfahren zur Herstellung von TMC entwickelt.

Beispielsweise kann die Herstellung von TMC durch Oxidation von Trimethylphenol (nachstehend als TMP bezeichnet), an einem homogenen Cobaltkomplex-Katalysator erfolgen (Salcomin-Verfahren). Die Oxidation erfolgt durch Einleiten von Sauerstoff beziehungsweise Luft in eine TMP, Katalysator und Lösungsmittel enthaltende Lösung. Die Aufarbeitung umfaßt in der Regel die Abtrennung von TMC aus der Reaktionsmischung durch Extraktion, Wasserdampfdestillation oder durch Dünnschichtverdampfung und sich anschließende Destillationen. Nichtumgesetztes TMP läßt sich durch diese Maßnahmen jedoch nicht vollständig entfernen, so daß bei der Reaktion auf möglichst vollständige Umsätze zu achten ist.

Auch andere Verfahren gehen bei der Herstellung von TMC von den Edukten TMP und Sauerstoff aus.

Dabei kann die Oxidation beispielsweise in Gegenwart eines homogenen Katalysatorsystems, das Kupfer- und Halogen-Ionen enthält, in einem einphasigen Lösungsmittelsystem erfolgen. Für die Aufarbeitung des anfallenden Reaktionsgemischs werden bevorzugt Extraktions- und Destillationsverfahren eingesetzt.

Die Oxidation von TMP zu TMC kann auch in einem zweiphasigen Lösungsmittelsystem durchgeführt werden, wobei der Vorteil dieses Verfahrens in der einfachen Rückführbarkeit des Katalysators besteht. Das dabei zugrundeliegende Reaktionssystem ist dreiphasig. Phase I ist die organische Phase, die das Lösungsmittel für TMP, TMC und für organische Zwischenprodukte der Oxidation enthält. Als organisches Lösungsmittel dienen dabei insbesondere Alkohole Phase II ist die wäßrige Phase, die den Großteil (d.h. in der Regel mehr als 90 %, bevorzugt mehr als 99 %) des gelösten Katalysatos enthält und mit Phase I nicht mischbar ist. Als Katalysatoren eignen sich insbesondere Kupferverbindungen enthaltende Systeme. Phase III ist die Gasphase, die das Edukt Sauerstoff enthält. Katalysator und Edukte befinden sich somit in verschiedenen Phasen - das Produkt TMC liegt, wie vorstehend erwähnt, zusammen mit TMP in der organischen Phase vor. Dies erleichtert die Abtrennung beziehungsweise die Rückführung des sich vorwiegend in der wäßrigen Phase befindlichen Katalysators von der organischen Produktmischung. Nach Scheidung der Phasen werden in einem Extraktionsverfahren die in der organischen Phase gelösten Katalysatorreste in der Regel vollständig oder nahezu vollständig entfernt und in den Oxidationsprozeß zurückgeführt. Zur weiteren Aufarbeitung der organischen Phase schließen sich bevorzugt mehrere Destillationsstufen an. Nichtumgesetztes TMP läßt sich dabei nicht vollständig, d.h. nur unter unwirtschaftlichen Bedingungen nahezu vollständig, von TMC abtrennen. Die Konsequenz ist, daß bis zu fast ca. 100 % Umsatz oxidiert werden muß, um TMP-Verunreinigungen im Produkt zu umgehen. Es ergeben sich lange Verweilzeiten im Oxidationsreaktor, da für die Umsetzung restlicher Anteile TMP (aufgrund der sich zum Ende der Reaktion erniedrigenden Reaktionsgeschwindigkeit) verhältnismäßig lange Reaktionszeiten in Kauf genommen werden müssen. Lange Verweilzeiten bedeuten geringe Raum-Zeit-Ausbeuten und somit wirtschaftliche Einbußen. Ein weiterer Nachteil besteht darin, daß bei der destillativen Aufarbeitung darauf geachtet werden muß, daß möglichst keine TMC-Zersetzung eintritt. Durch Destillation bei Temperaturen unterhalb von 120 °C kann die TMC-Zersetzung eingeschränkt werden.

Bei der mit CuCl/LiCl katalysierten Oxidation wird zum Beispiel das chlorierte Trimethylphenol (nachstehend als TMP-Cl bezeichnet) intermediär als Zwischenprodukt der Oxidation gebildet. Entsprechend der Abtrennung des TMP läßt sich auch TMP-Cl mit der vorstehend beschriebenen Aufarbeitung nur unvollständig vom Produkt entfernen. Dies gilt in gleicher Weise für viele der entstehenden Nebenprodukte. Nebenprodukte sind zum Beispiel Isomere des Hexamethylbisphenols sowie chlorierte Derivate davon, Epoxide, Ketale und verschiedene chlororganische Verbindungen. Letztere sind besonders problematisch, wenn diese nach der Aufarbeitung als Verunreinigung im TMC enthalten sind. Die Zersetzung der chlororganischen Verbindungen unter Freisetzung von HCl führt in nachfolgenden Reaktionsstufen zu Korrosionsproblemen. Außerdem wird der Hydrierungskatalysator des TMC (die nächste Stufe bei der Vitamin E-Herstellung ist die Hydrierung von TMC), z.B. ein Ni- oder Pd-Kontakt, durch entstehendes HCl vergiftet.

Die EP-B-0 216 351 schlägt daher zur Aufarbeitung die Behandlung der Oxidationsreaktionsmischung bei einer Temperatur von 120 °C bis 150 °C mit einer wäßrigen Alkalilösung vor, wobei dabei chlorierte Verbindungen entfernt werden. Diese Basenextraktion geht stets mit TMC-Zersetzung einher. Ein weiterer Nachteil ist, daß sich das Verfahren nicht zur vollständigen Entfernung von chlororganischen Verbindungen eignet (gemäß einer elementaranalytischen Untersuchung konnte beispielsweise der Chloranteil einer Oxidationsreaktionsmischung nur von ca. 1000 ppm auf ca. 300 ppm reduziert werden).

Der Erfindung lag die Aufgabe zugrunde, ein Verfahren bereitzustellen, bei dem TMC von Verunreinigungen befreit wird, beziehungsweise TMC aus TMC enthaltenden Mischungen isoliert wird. Von besonderer Bedeutung ist, daß (falls enthalten) dabei das Edukt TMP, Nebenprodukte sowie Zwischenprodukte der katalytischen Oxidation von TMP zu TMC zuverlässig abgetrennt werden. Die thermische Belastung, die von dem Verfahren hervorgerufen wird, sollte zur Vermeidung der TMC-Zersetzung gering sein (Temperaturen unter 50 °C wären dabei vorteilhaft).

Die Lösung dieser Aufgabe geht aus von dem Verfahren zur Reinigung von TMC oder zur Isolierung von TMC aus einem TMC enthaltenden Gemisch. Das erfindungsgemäße Verfahren ist dadurch gekennzeichnet, daß TMC durch Kristallisation abgeschieden wird.

Außerdem wird erfindungsgemäß auch ein Verfahren zur Herstellung von TMC durch katalytische Oxidation von TMP mit Sauerstoff bereitgestellt. Dabei entsteht ein Gemisch, das neben TMC außerdem TMP und/oder mindestens eine Komponente aus der Gruppe Lösungsmittel, Katalysatorreste, Nebenprodukte und Zwischenprodukte der katalytischen Oxidation von TMP zu TMC enthält. Das erfindungsgemäße Herstellungsverfahren ist dadurch gekennzeichnet, daß bei der ein oder mehrere Verfahrensschritte enthaltenden Aufarbeitung des Gemisches zumindest das vorstehend beschriebene Verfahren zur Reinigung von TMC und/oder zur Isolierung desselben aus einem TMC enthaltenden Gemisch durchgeführt wird.
Das TMC enthaltende Gemisch resultiert dabei in der Regel aus der katalytischen Oxidation von TMP. Bevorzugt wird dabei mit Sauerstoff oxidiert.

Die Kristallisation erfolgt bevorzugt erst nach ein oder mehreren Aufarbeitungsschritten in Form von Extraktionen und/oder Destillationen. Dabei wird in der Regel der Katalysator zu Beginn extraktiv entfernt, wobei anschließend Destillationsschritte folgen. Es ist vorteilhaft, das Medium, aus dem kristallisiert werden soll und in vielen Fällen noch zu einem Großteil das verwendete Lösungsmittel enthält, vor der Kristallisation destillativ einzuengen. Das TMC enthaltende Gemisch oder das zu reinigende TMC weist bei der Kristallisation in der Regel 1 bis 99,999 Gew.-%, bevorzugt 90 bis 99,999 Gew.-% TMC auf. Vorliegend wird zwischen Reinigung und Isolierung von TMC differenziert, da man bei geringem Anteil an Verunreinigungen (zum Beispiel 0,1 Gew.-% Verunreinigungen und 99,9 Gew.-% TMC) nur schwer von einer Isolierung des TMC sprechen kann. Die Befreiung von kleinen Anteilen an Verunreinigungen spielt insbesondere bei der Entfernung von chlorierten Verbindungen eine Rolle, da diese auch in geringen Konzentrationen (ca. im Bereich von 100 ppm), wie einleitend beschrieben, störend wirken. Eine Bereitstellung von reinem TMC ist auch deshalb von Bedeutung, da auf diese Weise gegebenenfalls auf die Reinigung des Folgeprodukts, Trimethylhydrochinon, verzichtet werden kann.

Durch die Kristallisation kann TMC von Zwischenprodukten und/oder Nebenprodukten, die bei der katalytischen Oxidation von TMP entstehen, gereinigt werden.

Auch nichtumgesetztes TMP kann durch Kristallisation des TMC abgetrennt werden. Die Durchführung der Oxidation bis zu einem Umsatz von nahe 100 % ist daher nicht mehr notwendig. Wie vorstehend erläutert ist eine nichtvollständige Umsetzung des TMC hinsichtlich der Raum-Zeit-Ausbeute günstig und wirkt sich deshalb positiv auf die Wirtschaftlichkeit des Verfahrens aus.

Die Kristallisation kann auch in Anwesenheit eines Hilfsstoffes, der prinzipiell jeder flüssige Stoff, beispielsweise ein Alkohol, sein kann, vorgenommen werden.

Ist der Anteil des Hilfsstoffes in der Mischung entsprechend hoch, so liegt anstatt einer Schmelze entweder eine Lösung oder eine Dispersion vor.

Die Kristallisation kann aus einer Lösung, Dispersion und/oder aus einer Schmelze erfolgen. Erfolgt die Kristallisation aus einer Schmelze, die nur TMP und TMC enthält, so liegt bezüglich dieses Binärsystems ein Eutektikum bei ca. 39 Gew.-% TMP, 61 Gew.-% TMC und einer Temperatur von ca. - 6 °C vor. Durch die Daten dieses eutektischen Punktes kann einerseits die sinnvolle Untergrenze der TMC-Konzentration von ca. 60 % vorgegeben werden und andererseits die minimale Kristallisationstemperatur von etwa -6°C angegeben werden, falls ausschließlich TMC und TMP in der Schmelze enthalten sind. Die maximale Kristallisationstemperatur wird durch den Schmelzpunkt von TMC vorgegeben, der bei ca. 32 °C liegt. Der bevorzugte Temperaturbereich für die Durchführung der Kristallisation beträgt somit in der Regel - 6 °C bis 32 °C.

Die eutonische Temperatur fällt mit steigendem Hilfsstoffgehalt, so daß TMC bei Hilfsstoff-haltigen Stoffmischungen auch unterhalb von - 6 °C unter Abtrennung von TMP kristallisiert werden kann.

Bei der Verwendung von niedrigsiedenden Alkoholen wie Methanol, Ethanol, Propanol oder Butanol als Hilfsstoff (diese Alkohole können bei der Oxidation von TMP zu TMC als Lösungsmittel eingesetzt werden und müssen in diesem Fall nicht mehr nachträglich zugegeben werden) liegt die Siedetemperatur des entsprechenden Systems häufig unterhalb des Schmelzpunktes von TMC. Die adiabate Verdampfung (in der Regel im Vakuum) ein oder mehrerer im zu kristallisierenden Medium enthaltenen Hilfsstoffe kann dann sinnvollerweise dazu genutzt werden, um das zu kristallisierende Medium auf Kristallisationstemperatur abzukühlen. Durch die Nutzung der adiabaten Verdampfung von Hilfsstoffen, besteht die Möglichkeit, auf einen verkrustungsgefährdeten Wärmetauscher zu verzichten.

Prinzipiell kann TMC auch aus einem Medium kristallisiert werden, das im wesentlichen Hilfsstoffe, Zwischenprodukte und/oder Nebenprodukte enthält. In einem solchen Fall sollte jedoch aufgrund der Wirtschaftlichkeit des Verfahrens bevorzugt zumindest 1% TMC in der entsprechenden Mischung enthalten sein.

Das verwendete Kristallisationsverfahren unterliegt keiner Beschränkung. Die Kristallisation kann kontinuierlich oder diskontinuierlich, einstufig oder mehrstufig durchgeführt werden.

Vorzugsweise erfolgt die Kristallisation einstufig. In einer anderen bevorzugten Ausführungsform der Erfindung wird die Kristallisation als fraktionierte Kristallisation durchgeführt.

Üblicherweise werden bei fraktionierter Kristallisation alle Stufen, die ein Kristallisat erzeugen, das reiner als die zugeführte Mischung ist, Reinigungsstufen und alle anderen Stufen Abtriebsstufen genannt. Zweckmäßigerweise werden mehrstufige Verfahren hierbei nach dem Gegenstromprinzip betrieben, bei dem nach der Kristallisation in jeder Stufe das Kristallisat von der Mutterlauge abgetrennt wird und dieses Kristallisat der jeweiligen Stufe mit dem nächsthöheren Reinheitsgrad zugeführt wird, während der Kristallisationsrückstand der jeweiligen Stufe mit dem nächstniedrigen Reinheitsgrad zugeführt wird.

Die Kristallisation erfolgt in einer vorteilhaften Ausführung der Erfindung als Suspensionskristallisation, bei der die einzelnen Kristalle dispers in der Mutterlauge vorliegen und die Suspension (Kristalle + Mutterlauge) durch Rühren oder Umwälzen bewegt wird. Bei der Kristallisation durch indirekte Kühlung (Kühlung der Suspension über Wärmeübertragungsflächen) kann die Wärme über Kratzkühler, die bevorzugt mit einem Rührkessel oder einem Behälter ohne Rührwerk verbunden sind, abgeführt werden.

Der Umlauf der Kristallsuspension wird hierbei durch eine Pumpe gewährleistet. Daneben besteht auch die Möglichkeit, die Wärme über die Wand eines Rührkessels mit wandgängigem Rührer abzuführen. Eine weitere bevorzugte Ausführungsform bei der indirekten Kühlungskristallisation ist die Verwendung von Kühlscheibenkristallisatoren. Bei einer weiteren geeigneten Variante zur Kristallisation durch indirekte Kühlung wird die Wärme über herkömmliche Wärmeüberträger (bevorzugt Rohrbündel- oder Plattenwärmeüberträger) abgeführt. Diese Apparate besitzen im Gegensatz zu Kratzkühlern, Rührkesseln mit wandgängigen Rührern oder Kühlkristallscheiben keine Vorrichtung zur Vermeidung von Kristallschichten auf den wärmeübertragenden Flächen. Wird im Betrieb ein Zustand erreicht, bei dem der Wärmedurchgangswiderstand durch Kristallschichtbildung einen zu hohen Wert annimmt, erfolgt die Umschaltung auf einen zweiten Apparat. Während der Betriebszeit des zweiten Apparats wird der erste Apparat regeneriert (vorzugsweise durch Abschmelzen der Kristallschicht oder Durchspülen des Apparats mit ungesättigter Lösung). Wird im zweiten Apparat ein zu hoher Wärmedurchgangswiderstand erreicht, schaltet man wieder auf den ersten Apparat um usw.. Diese Variante kann auch mit mehr als zwei Apparaten im Wechsel betrieben werden.

In einer weiteren vorteilhaften Ausführung der Erfindung erfolgt die Kristallisation in Apparaten, in denen die Kristalle im Kristallisationsapparat an gekühlten Flächen aufwachsen, d.h. im Apparat fixiert sind, z.B. Schichtkristallisationsverfahren oder statische Kristallisationsverfahren. Neben statischen Kristallisationsverfahren können erfindungsgemäß auch dynamische Kristallisationsverfahren durchgeführt werden, bei denen, im Gegensatz zum statischen Verfahren, aus bewegten Flüssigkeiten kristallisiert wird. Dabei können die entsprechenden Medien beispielsweise gerührt werden.

Zur Abtrennung der Mutterlauge von dem auskristallisierten TMC eignen sich alle bekannten Verfahren der Fest-Flüssig-Trennung. In einer bevorzugten Ausführungsform der Erfindung werden die Kristalle durch Filtrieren und/oder Zentrifugieren von der Mutterlauge abgetrennt. Vorteilhafterweise wird dem Filtrieren oder Zentrifugieren eine Voreindickung der Suspension, zum Beispiel durch Hydrozyklone, vorgeschaltet. Zum Zentrifugieren eignen sich alle bekannten Zentrifugen, die diskontinuierlich oder kontinuierlich arbeiten. Am vorteilhaftesten werden Schubzentrifugen verwendet, die ein- oder mehrstufig betrieben werden können. Daneben eignen sich auch Schneckensiebzentrifugen oder Schneckenaustragszentrifugen (Dekanter). Eine Filtration erfolgt vorteilhafterweise mittels Filternutschen, die diskontinuierlich oder kontinuierlich, mit oder ohne Rührwerk, oder mittels Bandfilter betrieben werden. Allgemein kann das Filtrieren unter Druck oder im Vakuum erfolgen.

Während und/oder nach der Fest-Flüssig-Trennung können weitere Verfahrensschritte zur Steigerung der Reinheit der Kristalle beziehungsweise des Kristallkuchens vorgesehen werden. In einer vorteilhaften Ausgestaltung der Erfindung werden nach der Abführung des Mediums, aus dem kristallisiert wurde, ein oder mehrere Wasch- und/oder Schwitzschritte mit den erhaltenen Kristallen durchgeführt.

Beim Waschen liegt die Waschflüssigkeitsmenge geeigneterweise zwischen 0,1 und 500 g Waschflüssigkeit/100 g Kristallisat, vorzugsweise zwischen 30 und 200 g Waschflüssigkeit/100 g Kristallisat. Geeignete Waschflüssigkeiten sind beispielsweise Hilfsstoffe (zum Beispiel Lösungsmittel) oder reines TMC. Das Waschen kann in hierfür üblichen Apparaten erfolgen. Vorteilhafterweise werden Waschkolonnen, in denen die Abtrennung der Mutterlauge und das Waschen in einem Apparat erfolgen, Zentrifugen, die ein- oder mehrstufig betrieben werden können, Filternutschen oder Bandfilter verwendet. Das Waschen kann auf Zentrifugen oder Bandfiltern ein- oder mehrstufig durchgeführt werden. Hierbei kann die Waschflüssigkeit im Gegenstrom zum Kristallkuchen geführt werden. Beim Schwitzen handelt es sich um ein lokales Abschmelzen verunreinigter Bereiche. Vorteilhafterweise beträgt die Schwitzmenge 0,1 bis 90 g abgeschmolzenes Kristallisat/100 g Kristallisat vor dem Schwitzen, vorzugsweise 5 bis 35 g abgeschmolzenes Kristallisat/100 g Kristallisat. Besonders bevorzugt ist die Durchführung des Schwitzens auf Zentrifugen oder Bandfiltern. Auch die Durchführung einer Kombination aus Waschen und Schwitzen in einem Apparat kann geeignet sein.

Im folgenden wird die Reinigung von TMC beziehungsweise die Isolierung von TMC gemäß dem erfindungsgemäßen Verfahren anhand von Ausführungsbeispielen näher beschrieben.

### Beispiel 1 (dynamische Schichtkristallisation)

TMP wird durch katalytische Oxidation mit Sauerstoff zu TMC umgesetzt. Dabei wird Bissalicylidenethylendiimin-kobalt (II) (Salcomin) als Katalysator und Dimethylformamid (DMF) als Lösungsmittel eingesetzt. Das Oxidationsreaktionsgemisch wird durch mehrstufige destillative Aufarbeitung vom Katalysator sowie von einem Großteil des Lösungsmittels (DMF) befreit. Dieses so erhaltene Gemisch ist das Medium, aus dem TMC kristallisiert werden soll. Die Zusammensetzung dieses Ausgangsgemisches ist in der nachstehenden Tabelle aufgeführt.

### Kristallisation:

Das Ausgangsgemisch wird mit einem Blattrührer (500 Umdrehungen pro Minute) in einem zylindrischen Behälter (Innendurchmesser I = 8 cm) bewegt. Die Kühlung erfolgt an einer ebenen Bodenplatte, die mit Hilfe eines Kühlmediums temperiert wird. Die Temperatur des Kühlmediums wird während der Kristallisation linear erniedrigt. Ausgehend von einer Temperatur des Kühlmediums von ca. 30 °C wird bei drei verschiedenen Versuchsvariationen mit jeweils unterschiedlicher Geschwindigkeit abgekühlt (Kühlraten siehe nachstehende Tabelle). Auf der Bodenplatte wird eine ca. 0,5 bis 1 cm dicke Kristallschicht ausgefroren. Anschließend wird die Apparatur um 180 °C gedreht, so daß die Flüssigkeit von der Kristallschicht abfließt. Letzte wird von der demontierten Bodenplatte abgenommen. Das Kristallisat enthält je nach Kühlrate zwischen ca. 99,4 Gew.-% und 99,8 Gew.-% TMC, wobei das entsprechende Ausgangsgemisch ca. 94,1 Gew.-% TMC aufgewiesen hat (genaue Daten sind in der nachstehenden Tabelle aufgeführt). TMP wird unabhängig von der Kühlrate vollständig vom Produkt entfernt. Die Abtrennung des Lösungsmittels DMF ist bei langsamer Abkühlung effektiver.

| | **Ausgangsgemisch** | **Kristallisat bei Kühlrate** | | |
|---|---|---|---|---|
| | | -12K/h | -30K/h | -60K/h |
| Masse | 300 g | 34,9 g | 26,1 g | 25,0 g |

| **enthaltene Komponenten** | **Zusammensetzung** | **Zusammensetzung** | **Zusammensetzung** | **Zusammensetzung** |
|---|---|---|---|---|
| TMC | 94,06 Gew.-% | 99,78 Gew.-% | 99,64 Gew.-% | 99,36 Gew.-% |
| TMP | 0,04 Gew.-% | 0 Gew.-% | 0 Gew.-% | 0 Gew.-% |
| DMF | 5,28 Gew.-% | 0,15 Gew.-% | 0,3 Gew.-% | 0,6 Gew.-% |
| MS | 0,45 Gew.-% | 0 Gew.-% | 0 Gew.-% | 0 Gew.-% |
| Epoxide | 0,12 Gew.-% | <0,01 Gew.-% | <0,01 Gew.-% | <0,01 Gew.-% |
| MS = Mittelsieder (ca. 6 unbestimmte Nebenprodukte) | | | | |

### Beispiel 2 (statische Schichtkristallisation)

TMP wird durch katalytische Oxidation mit Sauerstoff zu TMC umgesetzt. Als Lösungsmittel wird ein vorwiegend aus Wasser und 2-Ethylhexanol (2-EH) bestehendes zweiphasiges System eingesetzt, wobei ein Cu(II)-haltiger Katalysator vorwiegend in der wäßrigen Phase (in gelöster Form) vorliegt. Durch wäßrige Extraktion und anschließende destillative Aufarbeitung wird die sich abscheidende organische Phase des erhaltenden Oxidationsreaktionsgemischs vom Katalysator sowie von einem Großteil des Lösungsmittels 2-Ethylhexanol befreit. Das so erhaltene Gemisch ist das Medium, aus dem kristallisiert werden soll.

Es liegen zwei Kristallisationsversuche (als A und B bezeichnet), denen jeweils unterschiedliche Zusammensetzungen dieses Ausgangsgemischs zugrundeliegen, vor (siehe nachstehende Tabellen).

### Kristallisationsversuch A

Das ruhende Ausgangsgemisch wird in einem Doppelmantel-Glasrohr (Länge L = 1 m, Innendurchmesser D = 3 cm) gekühlt. Ein umgebendes Kühlmedium wird bei konstanter Kühlrate von - 0,6 K/h von 25 °C auf 18 °C temperiert und es bildet sich während dieser Zeit eine Kristallmasse. Das verbleibende flüssige Medium wird anschließend bei einer Temperatur von 18 °C über einen Bodenablaß von der Kristallmasse entfernt (Dauer dieses Vorgangs ca. 1 h). Anschließend erfolgt ein Schwitzschritt, bei dem die Wärmeträgertemperatur von 18 °C auf 30 °C erhöht wird (bei konstanter Erwärmung von 2 K/h) und die Schwitzfraktion über den Bodenablaß abläuft.

Die verbleibende Kristallfraktion wird schließlich durch Abschmelzen bei 40 °C aus dem Glasrohr entfernt.

| **Daten zu Kristallisationsversuch A** | | | | |
|---|---|---|---|---|
| | **Ausgangsgemisch** | **von der Kristallfraktion entferntes flüssiges Medium** | **Schwitzfraktion** | **Kristallfraktion** |
| Masse | 577,6 g | 184,4 g | 160,4 g | 232,8 g |

| **enthaltene Komponenten** | **Zusammensetzung** | | | **Zusammensetzung** |
|---|---|---|---|---|
| TMC | 95,4 Gew.-% | | | 99,94 Gew.-% |
| NK"A" | 0,22 Gew.-% | | | 0 Gew.-% |
| 2-EH | 3,04 Gew.-% | | | 0,06 Gew.-% |
| Ges-Cl in ppm | 940 | | | 42 |
| NK"A" = Nebenprodukt mit nichtidentifizierter Struktur Ges-Cl = Chlorgehalt (bestimmt durch Elementaranalyse) | | | | |

### Kristallisationsversuch B

Das ruhende Ausgangsgemisch wird in einem Doppelmantel-Glasrohr (Länge L = 1 m, Innendurchmesser D = 3 cm) gekühlt. Ein umgebendes Kühlmedium wird bei konstanter Kühlrate von - 0,25 K/h von 29 °C auf 26 °C temperiert und es bildet sich während dieser Zeit eine Kristallmasse. Das verbleibende flüssige Medium wird anschließend bei einer Temperatur von 26 °C über einen Bodenablaß von der Kristallmasse abgeführt (Dauer dieses Vorgangs ca. 1 h). Anschließend erfolgt ein Schwitzschritt, bei dem die Wärmeträgertemperatur von 26 °C auf 31 °C erhöht wird (bei konstanter Erwärmung von 0,9 K/h) ) und die Schwitzfraktion über den Bodenablaß abläuft. Die verbleibende Kristallfraktion wird schließlich durch Abschmelzen bei 40 °C aus dem Glasrohr entfernt.

| **Daten zu Kristallisationsversuch B** | | | | |
|---|---|---|---|---|
| | **Ausgangsgemisch** | **von der Kristallfraktionabgeführtes flüssiges Medium** | **Schwitzfraktion** | **Kristallfraktion** |
| Masse | 517,9 g | 140,1 g | 194,8 g | 183 |

| **enthaltene Komponenten** | **Zusammensetzung** | | | **Zusammensetzung** |
|---|---|---|---|---|
| TMC | 99,77 Gew.-% | | | 100 Gew.-% |
| NK"A" | 0,01 Gew.-% | | | 0 Gew.-% |
| 2-EH | 0,18 Gew.-% | | | 0 Gew.-% |
| Ges-Cl in ppm | 88 | | | 10 |
| NK"A" = Nebenprodukt mit nichtidentifizierter Struktur Ges-Cl = Chlorgehalt (bestimmt durch Elementaranalyse) | | | | |

Die Kristallisationsversuche A und B zeigen deutlich, daß der Chlorgehalt im Produkt sehr stark reduziert werden kann (Versuch A: von 940 ppm auf 42 ppm - d.h. auf ca. 4,5 % des ursprünglichen Chlorgehaltes; Versuch B: von 88 ppm auf 10 ppm - d.h. auf ca. 11,4 % des ursprünglichen Chlorgehaltes). Insbesondere Versuch B zeigt, daß praktisch reines TMC hergestellt werden kann.

## Patentansprüche

1. Verfahren zur Reinigung von 2,3,5-Trimethyl-p-benzochinon (TMC) oder zur Isolierung von TMC aus einem TMC enthaltenden Gemisch, **dadurch gekennzeichnet, dass** TMC durch Kristallisation abgeschieden wird.

2. Verfahren nach Anspruch 1, **dadurch gekennzeichnet, dass** das TMC enthaltende Gemisch aus der katalytischen Oxidation von Trimethylphenol (TMP) resultiert.

3. Verfahren nach Anspruch 1, **dadurch gekennzeichnet, dass** TMC von Zwischenprodukten und/oder Nebenprodukten, die bei der katalytischen Oxidation von Trimethylphenol (TMP) zu TMC entstehen, gereinigt wird.

4. Verfahren nach einem der Ansprüche 1 bis 3, **dadurch gekennzeichnet, dass** das TMC enthaltende Gemisch oder das zu reinigende TMC 1 bis 99,999 Gew.-%, bevorzugt 90 bis 99,999 Gew.-% TMC aufweist.

5. Verfahren nach einem der Ansprüche 1 bis 4, **dadurch gekennzeichnet, dass** die Kristallisation in einem bevorzugten Temperaturbereich von -6°C bis 32°C durchgeführt wird.

6. Verfahren nach Anspruch 5, **dadurch gekennzeichnet, dass** ein oder mehrere Hilfsstoffe in dem zu kristallisierenden Medium enthalten sind, deren adiabate Verdampfung dazu genutzt wird, das zu kristallisierende Medium auf Kristallisationstemperatur abzukühlen.

7. Verfahren nach einem der Ansprüche 1 bis 6, **dadurch gekennzeichnet, dass** vor der Kristallisation ein oder mehrere Aufarbeitungsschritte in Form von Extraktionen und/oder Destillationen durchgeführt werden.

8. Verfahren nach einem der Ansprüche 1 bis 7, **dadurch gekennzeichnet, dass** die Kristallisation aus einer Lösung, Dispersion oder einer Schmelze erfolgt.

9. Verfahren nach einem der Ansprüche 1 bis 8, **dadurch gekennzeichnet, dass** nach der Abführung des Mediums, aus dem kristallisiert wurde, ein oder mehrere Wasch- und/oder Schwitzschritte mit den erhaltenden Kristallen durchgeführt werden.

10. Verfahren zur Herstellung von 2,3,5-Trimethyl-p-benzochinon (TMC) durch katalytische Oxidation von Trimethylphenol (TMP) mit Sauerstoff unter Entstehung eines Gemisches, das neben TMC außerdem TMP und/oder mindestens eine Komponente aus der Gruppe Lösungsmittel, Katalysatorreste, Nebenprodukte und Zwischenprodukte der katalytischen Oxidation von TMP zu TMC enthält, **dadurch gekennzeichnet**, das das Gemisch nach dem Verfahren gemäß Anspruch 1 aufgearbeitet wird.

## Claims

1. A process for purifying 2,3,5-trimethyl-p-benzoquinone (TMQ) or for isolating TMQ from a TMQ-containing mixture, wherein TMQ is separated out by crystallization.

2. A process as claimed in claim 1, wherein the TMQ-containing mixture results from the catalytic oxidation of trimethylphenol (TMP).

3. A process as claimed in claim 1, wherein TMQ is purified from intermediates and/or byproducts formed in the catalytic oxidation of trimethylphenol (TMP) to TMQ.

4. A process as claimed in any of claims 1 to 3, wherein the TMQ-containing mixture or the TMQ to be purified comprises from 1 to 99.999% by weight, preferably 90 to 99.999% by weight, TMQ.

5. A process as claimed in any of claims 1 to 4, wherein the crystallization is carried out in a preferred temperature range of from -6°C to 32°C.

6. A process as claimed in claim 5, wherein one or more auxiliaries are present in the medium to be crystallized, and the adiabatic evaporation thereof is utilized to cool the medium to be crystallized to the crystallization temperature.

7. A process as claimed in any of claims 1 to 6, wherein one or more workup steps in the form of extractions and/or distillations are carried out before the crystallization.

8. A process as claimed in any of claims 1 to 7, wherein the crystallization takes place from a solution, dispersion or melt.

9. A process as claimed in any of claims 1 to 8, wherein one or more washing and/or sweating steps are carried out with the resulting crystals after removal of the medium from which crystallization has taken place.

10. A process for preparing 2,3,5-trimethyl-p-benzoquinone (TMQ) by catalytic oxidation of trimethylphenol (TMP) with oxygen to form a mixture which, besides TMQ, also contains TMP and/or at least one component from the group of solvent, catalyst residues, byproducts and intermediates of the catalytic oxidation of TMP to TMQ, wherein the mixture is worked up by the process as claimed in claim 1.

## Revendications

1. Procédé pour purifier la 2,3,5-triméthyl-p-benzoquinone (TMC) ou pour isoler la TMC à partir d'un mélange en contenant, **caractérisé par le fait que** l'on provoque sa séparation par cristallisation.

2. Procédé selon la revendication 1, **caractérisé par le fait que** le mélange contenant la TMC est obtenu par oxydation catalytique du triméthylphénol (TMP).

3. Procédé selon la revendication 1, **caractérisé par le fait que** la TMC est débarrassée par la purification des produits intermédiaires et/ou produits d'accompagnement formés à l'oxydation catalytique du triméthylphénol (TMP) en TMC.

4. Procédé selon l'une des revendications 1 à 3, **caractérisé par le fait que** le mélange contenant la TMC ou la TMC à purifier contient jusqu'à 99,999 % en poids et de préférence de 90 à 99,999 % en poids de TMC.

5. Procédé selon l'une des revendications 1 à 4, **caractérisé par le fait que** la cristallisation est réalisée dans l'intervalle de température préféré allant de -6 à +32°C.

6. Procédé selon la revendication 5, **caractérisé par le fait que** le milieu de cristallisation contient un ou plusieurs produits auxiliaires dont on exploite la vaporisation adiabatique pour refroidir le milieu de cristallisation à la température de cristallisation.

7. Procédé selon l'une des revendications 1 à 6, **caractérisé par le fait que**, avant la cristallisation, on procède à un ou plusieurs traitements consistant en des extractions et/ou des distillations.

8. Procédé selon l'une des revendications 1 à 7, **caractérisé par le fait que** la cristallisation est réalisée à partir d'une solution, d'une dispersion ou d'une masse fondue.

9. Procédé selon l'une des revendications 1 à 8, **caractérisé par le fait que**, après évacuation du milieu duquel on a cristallisé, on procède sur les cristaux obtenus à une ou plusieurs opérations de lavage et/ou de ressuage.

10. Procédé pour la préparation de la 2,3,5-triméthyl-p-benzoquinone (TMC) par oxydation catalytique du triméthylphénol (TMP) à l'aide de l'oxygène avec formation d'un mélange qui, en plus de la TMC, contient de la TMP et/ou au moins un composant du groupe consistant en le solvant, les résidus de catalyseur, les produits d'accompagnement et les produits intermédiaires de l'oxydation catalytique du TMP en TMC, **caractérisé par le fait que** l'on traite le mélange par le procédé selon la revendication 1.
